# EUROPEAN PATENT APPLICATION

(11) **EP 0 884 590 A1**
(43) Date of publication of application: **16.12.1998**
(21) Application number: 97304102.3
(22) Date of filing: 12.06.1997
(51) Int. Cl.: G01N 31/22, G01N 21/78, G01N 33/00

(54) **Carbon monoxide sensors with controlled response threshold**

(71) Applicant: QUANTUM GROUP, San Diego, CA 92121 (US)
(72) Inventor: Goldstein, Mark K., Del Mar, California 92014 (US); Oum, Michelle S., San Diego, California 92105 (US); Johnson, Paula I., San Diego, California 92117 (US); Li, Ping, Temecula, California 92591 (US); Pucher, Shawn R., San Diego, California 92131 (US)
(74) Representative: Griffin, Kenneth David

(57) **Abstract**

Carbon monoxide sensors having a controlled response threshold comprise a porous semi-transparent substrate that is impregnated with a chemical regent. The chemical reagent is formulated to regenerate itself for a period of at least a year, and is tailored to provide optimum carbon monoxide response within a predetermined range of relative humidity conditions. In one embodiment of the invention, the chemical reagent is formulated to provide a predetermined carbon monoxide response threshold of greater than about 15 ppm. In another embodiment, the sensor comprises an optically thin metal oxide film interposed between the substrate surface and the chemical reagent. The metal oxide film is sufficiently thin to provide a predetermined carbon monoxide response threshold of greater than about 15 ppm. Carbon monoxide sensor systems comprise two of the sensors that are both specifically formulated to provide optimum carbon monoxide response under low to high relative humidity conditions, and that are each adapted to provide a carbon monoxide response threshold of greater than about 15 ppm.

## Description

### Copending Patent Applications

This application is a continuation-in-part of copending parent U.S. Patent Application Serial Number 08/297,141 filed on August 29, 1994.

This invention relates to carbon monoxide sensors and, more particularly, to chemoptical carbon monoxide sensors having a controlled response threshold.

Carbon monoxide (CO) is a colorless and odorless gas whose hazards to life are well known. Due to the dangers posed to humans from exposure to CO in automobiles, airplanes, industrial plants, mines, homes, and other environments in which humans are present for extended periods of time, several methods for the detection of CO have been developed including chemical reagent based sensors and solid state semiconductor based sensors.

Chemical reagent based sensors, for detecting the presence of CO have been known for many years. One of the first examples of such a sensor is described by Shepherd in Analytical Chemistry Vol. 19, No. 2, (1974) pages 77-81. Shepherd discloses a non-reversible CO sensing composition that uses a combination of palladium sulfate solution and ammonium molybdate solution impregnated into silica gel. Sensors made of this sensing composition can be used only once, since they do not self-regenerate, and have a very short lifetime (approximately 1 hour) when exposed to daily living conditions. In addition, the Shepherd sensors are expensive to use and delicate to handle.

K. Schuler and G. Schrauzer improved upon this technology by adding a third metallic salt component which produced a self-regenerating, short lived CO sensing composition. As disclosed in U.S. Patent Number 4,043,934, Schuler uses the CO sensing composition to produce sensors that are sensitive to low level concentrations of CO. However, while these sensors and the associated system are effective in detecting carbon monoxide, they have not met with commercial acceptance due to the short (1-3 months) functional life of the sensors when exposed to daily living conditions.

In U.S. Patent Number 5,063,164, Goldstein discloses a CO sensor that is useful in consumer products, and operates for at least one year when exposed to daily living conditions without maintenance or calibration. However, CO detectors containing sensors having the formulations disclosed by Goldstein do not pass Underwriter's Laboratories 2034 standards for carbon monoxide detectors effective October 1, 1995, especially with respect to response threshold to low level concentrations of CO. Specifically, the Goldstein sensors do not respond well to CO under conditions of high humidity and tend to respond too well to low level concentrations of CO. Therefore, the sensors disclosed in U.S. Patent Number 5,063,164 can be characterized as not having a controlled response threshold. i.e., being sensitive to CO at low concentration levels, and having an optimal sensitivity to CO at moderate to low relative humidity conditions.

U.S. Patent Numbers 5,302,350; 5,346,671; and, 5,405,583 issued to Goswami et al. describe another type of chemical based CO sensor having CO sensing chemical formulations that are similar to that of Schuler et al. and Goldstein previously discussed. In the first two patents, Goswami et al. teaches and discloses the details of the chemistry of a CO sensing solution. Goswami et al. discloses how the adjustment or addition of chemical reagents changes the response of the CO sensing solution to interfering agents (H₂S, NO₂, N₂O, propane, butane. etc.). The CO sensing formulation taught by Goswami comprises a catalyst (such as palladium chloride or palladium sulfate), a color forming agent (silicomolybdic acid), a reversing agent (iron, chromium or cerium salt), and a redox modifying agent (a source of acetate ion such as sodium acetate). Despite the detail of the disclosure, Goswami et al. fails to teach how to make a useful CO sensor. Further, the useful lifetime and the response threshold of the sensing solution to CO under varying conditions of humidity and temperature are not discussed.

In the third patent (Number 5,405,583), Goswami et al. discloses and teaches how to make a useful CO sensor using the CO sensing solution disclosed in the previous two patents. As taught by Goswami, a mixture of the CO sensing solution, an embedding matrix and a permeation enhancer are combined and coated or deposited onto a substrate. The embedding matrix is a film forming crosslinkable, polymerizable monomer or polymer and the permeation enhancer is a plasticizer. Substrates disclosed by Goswami et al. include optical fibers, glass shapes(rods, plates, prisms, etc..), integrated optical materials (zirconates, titanates, etc..), polymeric materials (plexiglass, polyimide, etc.), single crystals and, semiconductor materials. Schematically, Goswami et al. discloses a CO detecting apparatus that is made of a light source, such as a light emitting diode, a CO sensor, and a photodiode detector. Once again Goswami et al. fails to disclose or discuss the useful lifetime of the disclosed sensor or any other response characteristic of the sensors.

In co-pending parent application Serial Number 08/297,141 filed 29 August, 1994 Goldstein et al. discloses an improved CO sensor system that is responsive to CO under a wide range of humidity and temperature. The CO sensor system is made of two semitransparent, chemical reagent impregnated CO sensing disks that are optically aligned in a sensor housing. The first sensor disk is that disclosed by Goldstein, in U.S. Patent Number 5,063,164. The second sensor disk comprises a semitransparent substrate, with enlarged surface pores, impregnated with a modified CO sensing solution. The modified CO sensing solution contains, in addition to the previously disclosed Goldstein sensor formulation, a trifluorinated organic anion , an organic solvent, and a source of bromide ion. Due to the changes in both the sensing solution chemistry and the surface pore size of the substrate, the second sensor is more responsive to CO under conditions of high humidity.

Goldstein teaches the optical combination of a CO sensor that responds to CO under low humidity conditions with a CO sensor that responds to CO under high humidity conditions, thus generating a long life CO sensor system that responds to CO under a wide range of humidity conditions. However, Goldstein does not disclose or suggest a sensor system having a predetermined CO response threshold.

Despite the above noted advances in CO sensor technology, Underwriter's Laboratories (UL), and similar certification entities in Japan, and Europe have developed stringent standards requiring CO detectors not to respond to prescribed low levels of CO. Under UL's 2034 certification standard for CO detectors (effective October 1, 1995), a CO detector must not alarm when exposed to 15 ppm CO for 30 days. The current Japanese standard requires a CO detector not alarm upon exposure to CO at 50 ppm for living room applications and exposure to CO at 100 ppm for bathroom applications. The European standard is less stringent than the Japanese standard in that the CO detector must not alarm upon exposure to 45 ppm of CO for 60 minutes.

Therefore, it is desirable that a carbon monoxide detector be constructed having chemical based CO sensors which can be tailored to withstand the above-mentioned criteria of exposure to low levels of CO for extended periods of time and yet still be responsive to higher levels of CO under a wide humidity and temperature range. Further, it is desirable that the sensors used in such a system have a lifetime of at least three years upon exposure to normal daily living and operating conditions.

The present invention is directed to a carbon monoxide (CO) sensing system that is responsive to CO under a wide range of relative humidity and has a predetermined threshold response to carbon monoxide below which the sensor will not cause an alarm response. This is achieved by optically combining a sensor having an optimum response to CO under conditions of low to ambient relative humidity with a sensor having an optimum response to CO under conditions of ambient to high humidity.

Each sensor comprises of a porous, semi-transparent monolithic sensor substrate, such as porous silicon dioxide, impregnated with different aqueous chemical reagent mixtures. The humidity range and the response threshold of a sensor is controlled by the formulation of the chemical reagent mixture used to impregnate the sensor substrate, the chemical modification of the sensor substrate surface, or both.

Exemplary formulations of the chemical reagent mixture used to make sensors having a 35 ppm CO response threshold are given for both the low to ambient humidity sensor and the ambient to high humidity sensor. Experimental data shows that sensors made using these exemplary formulations do not trigger an alarm response when exposed to 35 ppm CO for more than 30 days. However, the same sensors rapidly darken, thereby triggering an alarm response upon being exposed to 100 ppm for 90 minutes.

It has been discovered that the application of a molecularly thin, optically transparent, coating of metal oxide onto the surface of the sensor substrate can also control the response threshold. Coatings of metal oxides, such as those selected from the group consisting of iron, copper, manganese, molybdenum, chromium, and mixtures thereof are useful in this respect. Impregnation of metal oxide treated sensor substrates with chemical reagent gives sensors that do not trigger an alarm response even afier 290 days exposure to 35 ppm CO. However, upon exposure to 100 ppm CO an alarm response is triggered within 90 minutes under a wide range of relative humidity conditions.
FIG. 1 is a schematic view of a CO sensing system incorporating CO sensors constructed according to principles of this invention;
FIG. 2 is a chart illustrating in graphical form the experimental data of Table 1;
FIG. 3 is a chart illustrating in graphical form the experimental data of Table 2;
FIG. 4 is a chart illustrating in graphical form the experimental data of Table 3;
FIG. 5 is a chart illustrating in graphical form the experimental data of Table 4; and
FIG. 6 is a chart illustrating in graphical form the experimental data of Table 11.

The following terms and words are used herein and are defined to mean the following:
"ambient relative humidity" means a relative humidity between about 45-55%;
"low relative humidity" means a relative humidity between about 15-35%;
"high relative humidity" means a relative humidity between about 80-90%;
"surface pore size" means the average diameter of the pores on the surface of the sensor substrate;
"response threshold" is the concentration level of CO below which a sensor will not cause an alarm response;
"ambient temperature" means a temperature between about 20-25°C.

The present invention comprises of an improved chemical sensor system for detecting the presence of carbon monoxide (CO) above predetermined threshold concentrations of CO (e.g. 15, 35, 45, 50, or 100 ppm). The chemical sensor system constructed according to principles of this invention is an improvement over the sensor system disclosed in U.S. Patent No. 5,063,164 and in the parent application in that the sensor system can be systematically modified to enable the tailoring of the response threshold of the sensor system to have a predetermined response threshold to low level concentrations of CO.

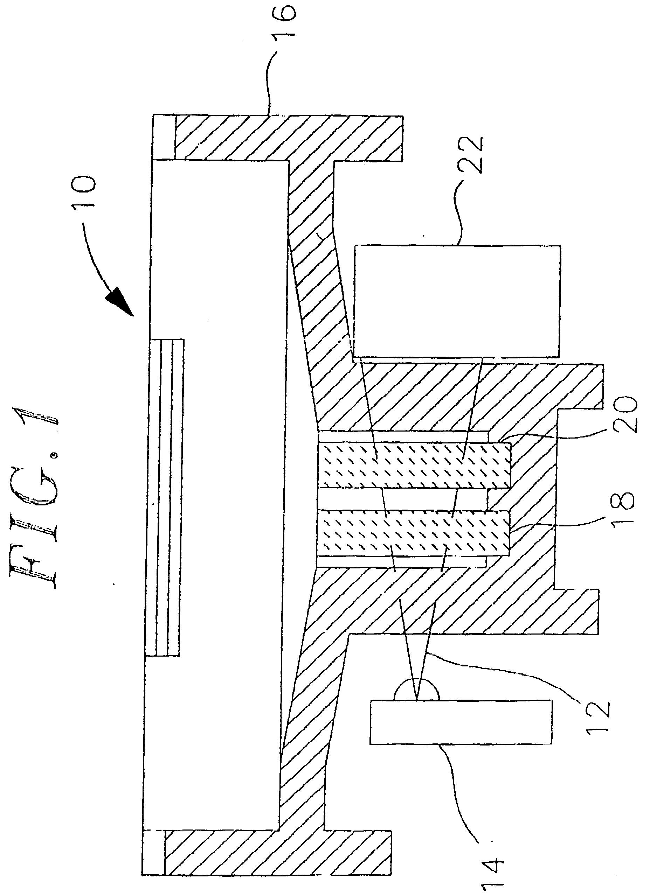

FIG. 1 illustrates a chemical sensor system 10 made according to principles of this invention. As schematically shown, light 12 is emitted by a light emitting means, such as an infrared light emitting diode (LED) 14, positioned adjacent to a sensor housing 16. The light 12 passes through a first sensor 18 and then a second sensor 20 contained within the sensor housing 16. The attenuated light is detected by a light detecting means, such as a photodiode 22 which is an integrated part of the electronic system of the CO detector (not shown). Upon exposure to CO, either one or both sensors darken, and the amount of light being detected by the photodiode correspondingly changes. The electronic system of the CO detector triggers a suitable alarm response, such as an audible alarm, flashing light or activation of a safety shut-off valve, based on either the change in the intensity of the light or the rate of change in the intensity of the light being detected by the photodiode.

Each of the sensors 18 and 20 in this invention are distinctly different self-regenerating sensors having different characteristic response ranges to CO. The first sensor 18 responds best to CO under low to ambient relative humidity and the second sensor 20 responds best to CO under ambient to high relative humidity. Each sensor is made by impregnating a porous, semi-transparent substrate with a chemical reagent. The mixture of chemical compounds used to make up the impregnating chemical reagent for the low-ambient humidity sensor is different from the mixture of chemical compounds used to make up the impregnating chemical reagent for the ambient-high humidity sensors. It has been discovered that the response threshold of the sensors can be controlled by modifying the formulation of the impregnating chemical reagent and/or by modifying the characteristics of the substrate , as is discussed below.

Accordingly, a sensor system comprising a first sensor that responds best to CO under conditions of low to ambient humidity and a second sensor that responds best to CO under conditions of ambient to high relative humidity are optically combined, thus enabling the sensor system to both monitor CO over the full range of relative humidities as required by most standards and also not respond to CO below predetermined low concentration levels. Although the present invention has been described as being made of two sensors, it should be understood that either a single sensor, portions of which are coated or treated differently or a combination of a plurality of sensors each having distinct response characteristics can be used to form a sensor system within the scope of this invention.

Control of the response threshold of the sensors upon exposure to CO can be generally brought about in two different ways. The first involves modifying the formula of the chemical reagent used to impregnate the substrate by adding or removing specific chemical compounds. The second involves modifying the substrate's surface characteristics and properties. The use of these methods, separately or in combination with each other, is understood to being within the scope of the present invention, although each is described separately below.

The following discussion of response threshold controlled sensors is focused primarily on sensors having a 35 ppm CO response threshold so that when they are combined together into a sensor system, the system will meet the UL 2034 standards in effect on October 1, 1995. However, given this disclosure one skilled in the art could apply the principles disclosed herein to obtain sensors and sensor systems that meet the Japanese and European standards. Therefore, we consider such modifications and formulations to be within the scope of the present invention.

We have discovered several different formulations of the chemical reagent used to impregnate the substrate that allow control over the response threshold of chemical reagent based CO sensors. Thus by combining "low" humidity and "high" humidity sensors, both having a predetermined response threshold, a sensor system is generated that responds under a wide range of conditions and is able to meet the UL 2034 specifications effective on October 1, 1995.

Controlling the threshold response of the sensor for low to ambient humidity conditions is accomplished by using two different chemical reagent formulations. Both formulations are related to the formulations disclosed in U.S. Patent No. 5,063,164,(hereafter referred to as "Formula'164") the contents of which are hereby incorporated by reference. Sensors made with the first chemical reagent formulation (hereafter referred to as Formula #1 sensors) appear to the eye to be no different from sensors made with Formula'164 (hereafter refereed to as "Formula'164 sensors"), in that they both are yellow and absorb light proportionally to the surrounding concentration of CO. However, a Formula #1 sensor is distinctly different in its response threshold to low level concentrations of CO. As shown below, unlike the 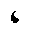 164 sensor, the Formula #1 sensor is able to withstand long term exposure to low level CO concentrations.

It should be noted at this point that an ertor in numerical units exists throughout U.S. Patent No. 5,063,164. Everywhere that the units of nanometers (1 nm = 10⁻⁹ meters) are placed after a number, the number should be written with the decimal place moved two places to the left. For example, a number written as 100 nanometers should actually read 1.0 nanometers. This should be taken into account when comparing/contrasting the improved sensor system in this patent application with the system described in 5,063,164.

The substrate used to form the Formula #1 sensor is a porous, semi-transparent material that is at least partially transparent to light. Desired substrate materials include, but not limited to commercial silica gel desiccants in bead form (available form most major suppliers of silica gel), porous silicon dioxide such as "GELSIL" (TM) available from Geltech of Alachua, Florida, and other similar porous, semi-transparent monolithic substrates. A variety of physical shapes and forms are obtainable by suitable commercial means, however disk-shaped pieces are preferred. The average surface pore size of the substrate can range from about 15 to 100 nanometers (1.5 x 10⁻⁸ - 1.0 x 10⁻⁷m) although a pore size within the range of from about 15 to 35 nanometers (1.5 x 10⁻⁸ - 3.5 x 10⁻⁸ m) is preferred, and a pore size of approximately 27 nanometers (2.7 x 10⁻⁸ m) is most preferred.

The Formula #1 chemical reagent solution used to impregnate the substrate is a mixture of chemical compounds and comprises at least one compound selected from each of the following Groups: Group 1 comprising palladium compounds and their hydrates; Group 2 comprising molybdenum compounds and their hydrates; Group 3 comprising copper salts and their hydrates; Group 4 comprising cyclodextrin molecular encapsulants; Group 5 comprising soluble chloride salts and their hydrates; and Group 6 comprising halogenated acetic acids, and alkali metal and alkaline-earth metal salts of halogenated acetates.

Preferred Group 1 materials include those selected from the group consisting of palladium chloride; palladium bromide; palladium sulfate; calcium, sodium and potassium salts of tetrachloropallidate, bromotrichloropallidate, dibromodichloropallidate, tribromochloropallidate and tetrabromopallidate ions; and mixtures thereof. Preferred Group 2 materials include those selected from the group consisting of silicomolybdic acid; salts of silicomolybdic acid: molybdenum trioxide; ammonium molybdate; alkali or alkaline earth salts of molybdate anion; and mixtures thereof. Preferred Group 3 materials include those selected from the group consisting of copper sulfate; copper nitrate; copper bromide; copper chloride; copper perchlorate; and mixtures thereof. Preferred Group 4 materials include those selected from the group consisting of α-cyclodextrin; β-cyclodextrin; modified β-cyclodextrin; γ-cyclodextrin; cyclodextrins having an internal cavity of at least 5 Å (5 x 10^{- 10} m); and mixtures thereof. Preferred Group 5 materials include chloride salts selected from the group consisting of sodium, lithium, magnesium, calcium, and strontium ions, and mixtures thereof. Preferred Group 6 materials include those selected from the group consisting of trichloroacetic acid; tribromoacetic acid; sodium, potassium, calcium salts of trichloroacetate; sodium, potassium, calcium salts of tribromoacetate; and mixtures thereof.

Components of the Formula #1 chemical reagent solution are present in the following mole ratio ranges to provide a chemical sensor having a desired controlled CO response threshold:
Group 2 : Group 1 = 0.051 to 1.269
Group 3 : Group 1 = 0.0094 to 0.235
Group 4 : Group 1 = 0.013 to 0.329
Group 5 : Group 1 = 0.573 to 13.42
Group 6 : Group 1 = 0.181 to 45.13

In a preferred embodiment of the Formula #1 chemical reagent solution, an aqueous solution is made wherein the compound from Group 1 is a mixture of palladium chloride and sodium tetrachloropallidate; the compound from Group 2 is silicomolybdic acid; the compound from Group 3 is copper chloride dihydrate; the compound from Group 4 is a mixture of β-cyclodextrin and γ-cyclodextrin; the compound from Group 5 is calcium chloride dihydrate and the compound from Group 6 is a mixture of trichloroacetic acid and sodium trichloroacetate.

Control of the response threshold in the Formula #1 sensor is a carefully adjusted balance of competing chemical reactions brought about by the adjustment of the molar ratios of compounds involved in those reactions. It has been found that the response threshold of a Formula #1 sensor can be increased by the addition of Group 3 compounds. However, the addition of Group 3 compounds retards the response of the sensor to high concentrations of CO (e.g. 100 ppm CO). In order to obtain a sensor with a predetermined response threshold that still rapidly responds to higher concentrations of CO, Group 6 compounds must also be added to the chemical reagent formulation. The addition of Group 6 compounds alone has been found to increase the response of the sensors to CO. Therefore in order to obtain a Formula #1 sensor with a predetermined threshold response, the desensitizing affects of Group 3 compounds must be counter balanced by the sensitizing affects of the Group 6 compounds. Even though the Formula #1 chemical reagent formulation appears to be the same as that disclosed in the parent application, the point of invention lies within the very different molar ratio of compounds, and the balance of competing chemical reactions thus resulting in a predetermined response threshold, as supported by the following illustrative specific embodiment. Accordingly, adherance to molar ratio ranges for the Groups of Formula #1 is key to the formulation of a sensor having both the predetermined CO response threshold and being capable of rapidly responding to higher CO concentrations.

The following example of a Formula #1 sensor with a CO response threshold of 35 ppm is intended to illustrate and clarify a specific embodiment of the present invention and is not intended to limit the scope of the invention which is defined by the claims. All chemical reagents used for this example and all other examples of the invention were of reagent grade and commonly available from a variety of commercial sources. All reactions in the examples of this invention were carried out using standard laboratory techniques and equipment under ambient atmospheric conditions unless otherwise noted.

### EXAMPLE #1 - Preparation of Formula #1 Sensors Having a CO Response Threshold of 35 ppm

100 porous, silica GELSIL(TM) discs (6.0 mm diameter, 2.9 mm thick) with a average surface pore size of 27.0 nm (2.70 x 10⁻⁸ m) available from Geltech of Alachua FL were soaked under ambient conditions for 24 hours in 40 ml of an aqueous chemical reagent solution containing the following: 0.0048 M sodium tetrachloropallidate (Group 1); 0.0384 M palladium chloride (Group 1); 0.011 M silicomolybdic acid (Group 2); 0.00204 M copper chloride dihydrate (Group 3); 0.00164 M β-cyclodextrin (Group 4); 0.00484 M γ-cyclodextrin (Group 4); 0.11616 M calcium chloride dihydrate (Group 5); 0.0799 M sodium trichloroacetate (Group 6); and, 0.3100 M trichloroacetic acid (Group 6). The sensors were removed from the solution and were air dried for 24 hours at room temperature, heated at 40°C for 24 hours and finally cooled to room temperature. Typically, sensors prepared in this manner are clear and have a yellow to amber appearance.

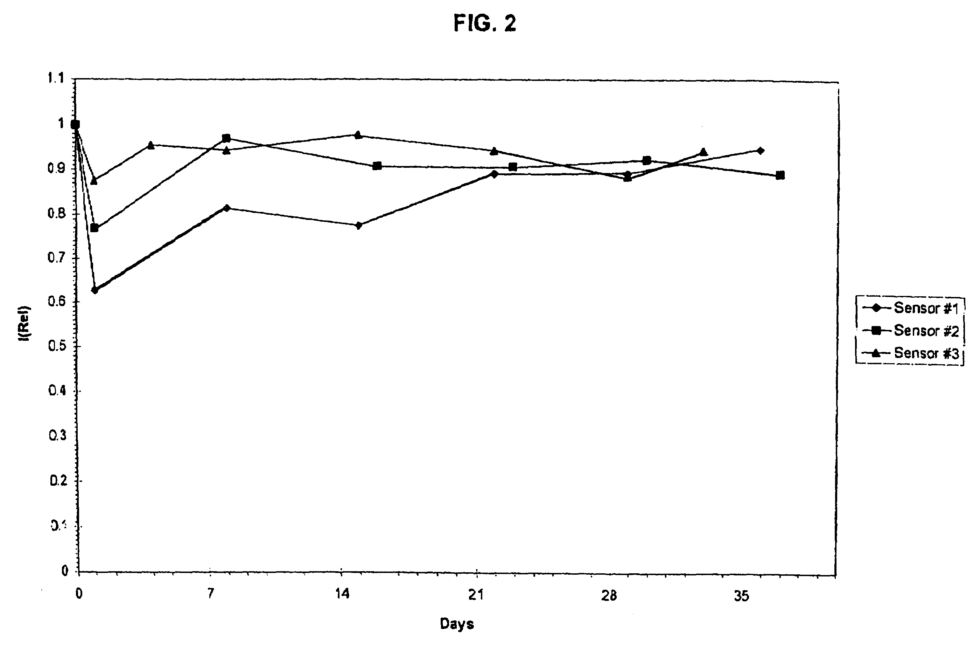

The response of Formula #1 sensors to 35 ppm CO at ambient relative humidity and ambient temperature is given in Table 1 and experimental data is shown in graphical form in FIG. 2. All values are given relative to the initial intensity (Io) of the light attenuated by the sensor.

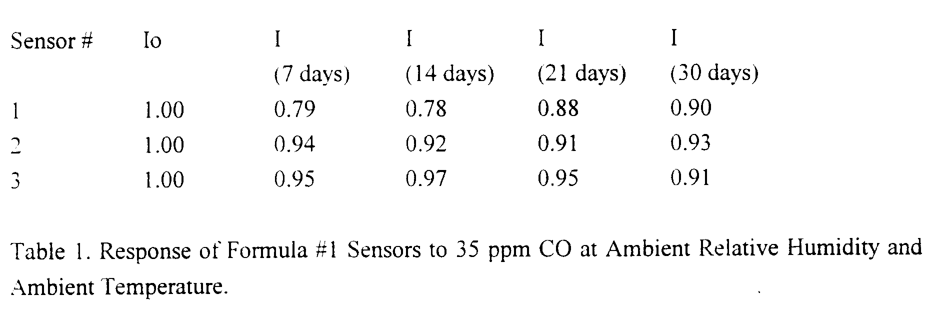

The above data shows that the Formula #1 sensor partially darkens during the 30 day exposure to 35 ppm CO. Even though the detected level of attenuated light varies over time, the intensity at any particular time is not reduced enough to trigger an alarm response in an electronic CO detector(e.g. I = 0.30 or less). In contrast, a '164 sensor exposed to the same conditions darkens to a level so that within 8 days an electronic CO detector triggers an alarm response.

Another chemical reagent formulation (hereafter referred to as "Formula #2) can be used to make sensors having an optimum response to CO under low to ambient conditions and a predetermined response threshold. The preferred sensor substrate is identical to that used above for Formula #1. The Formula #2 chemical reagent solution used to impregnate the substrate is a mixture of chemical compounds and comprises at least one compound selected from each of the following Groups: Group 1 comprising palladium compounds and their hydrates; Group 2 comprising molybdenum compounds and their hydrates; Group 3 comprising copper salts and their hydrates; Group 4 comprising cyclodextrin molecular encapsulants; and Group 5 comprising soluble chloride salts and their hydrates. Preferred Group 1 to 5 materials are the same as those previously described above for Formula #1 .

Components of the Formula #2 chemical reagent solution are present in the following mole ratio ranges to provide a chemical sensor having a desired controlled CO response threshold:
Group 2 : Group 1 = 0.15 to 0.43
Group 3 : Group 1 = 0.042 to 0.122
Group 4 : Group 1 = 0.039 to 0.111
Group 5 : Group 1 = 1.34 to 3.73

In a preferred embodiment of the Formula #2 chemical reagent solution, an aqueous solution is made wherein the compound from Group 1 is a mixture of palladium chloride and sodium tetrachloropallidate; the compound from Group 2 is silicomolybdic acid; the compound from Group 3 is copper chloride dihydrate; the compounds from Group 4 are a mixture of β-cyclodextrin and γ-cyclodextrin; and, the compound from Group 5 is calcium chloride dihydrate.

Although this formulation appears to be identical to that used for a '164 sensor, a point of invention lies within the different molar ratio of compounds and the resulting response threshold. As previously discussed, the adjustment of the threshold response is a balance between the chemical compounds used to adjust the response threshold and chemical compounds used to adjust the performance of the sensor at high CO concentrations. In order to upwardly adjust the response threshold of a Formula #2 sensor without adversely affecting the performance, the amounts of Group 2 and Group 3 compounds must be increased together. It is therefore key that the molar ratio ranges for the Groups described above be adhered to for forming the Formula #2 sensors. The following specific embodiment is given as an illustration of a Formula #2 sensor.

The following example of a Formula #2 sensor with a response threshold of 35 ppm is intended to illustrate and clarify one specific embodiment of the present invention and is not intended to limit the scope of the invention.

### EXAMPLE #2 - Preparation of Formula #2 Sensors Having a CO Response Threshold of 35 ppm

100 porous, silica GELSIL(TM) discs (6.0 mm diameter, 2.9 mm thick) with a average surface pore size of 27.0 nm (2.70 x 10⁻⁸ m) available from Geltech of Alachua FL were soaked under ambient conditions tor 24 hours in 40 ml of an aqueous chemical reagent solution containing the following: 0.0018 M sodium tetrachloropallidate (Group 1); 0.0153 M palladium chloride (Group 1); 0.0044 M silicomolybdic acid (Group 2); 0.0013 M copper chloride dihydrate (Group 3); 0.0006 M β-cyclodextrin (Group 4); 0.0005 M γ-cyclodextrin (Group 4); and, 0.0384 M calcium chloride dihydrate (Group 5). The sensors were removed from the solution and were air dried for 24 hours at room temperature, heated at 40°C for 24 hours and finally cooled to room temperature. Typically, sensors prepared in this manner are clear and have a yellow to amber appearance.

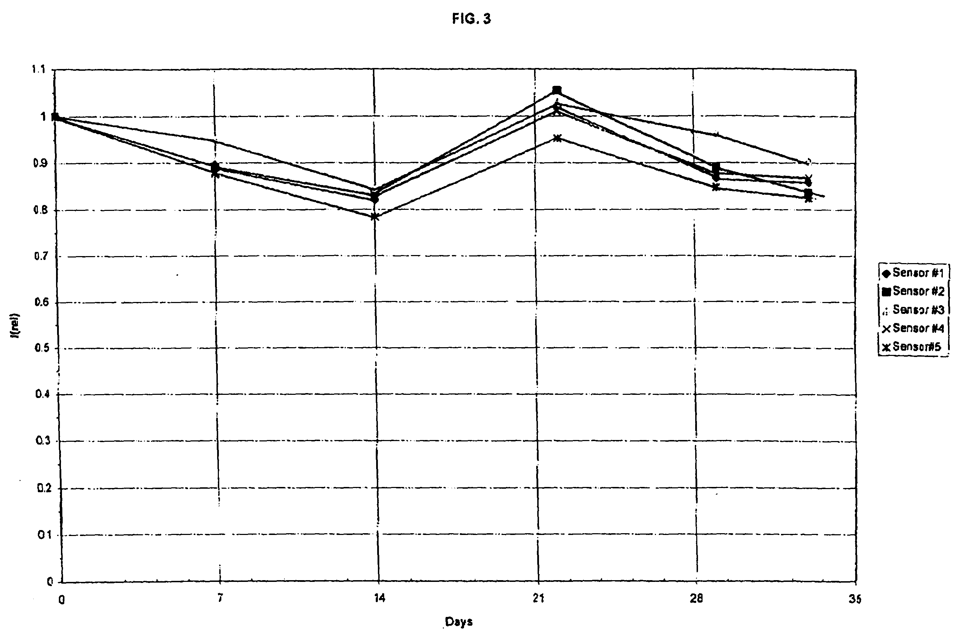

The response of Formula #2 sensors to 35 ppm CO at ambient relative humidity is given in Table 2 and experimental data is shown in graphical form in FIG. 3. All values are given relative to the initial intensity (Io) of the light attenuated by the sensor.

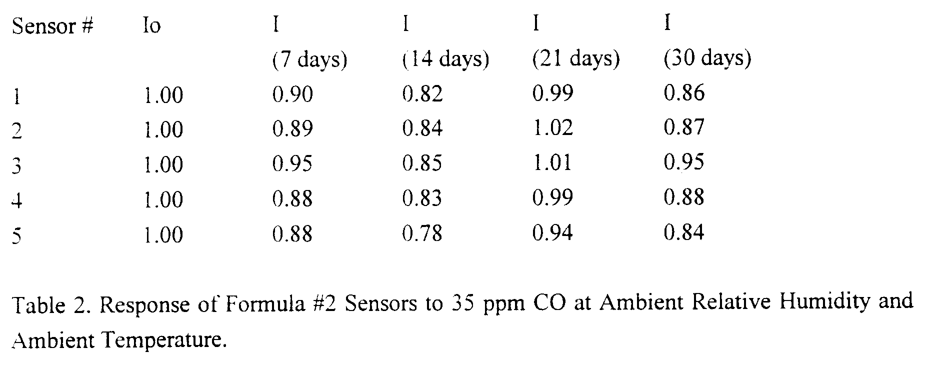

The above data shows that the Formula #2 sensor partially darkens during the exposure to 35 ppm CO. Even though the detected level of attenuated light varies over time, the intensity at any particular time is not reduced enough to trigger an alarm response in an electronic CO detector (e.g. I = 0.30 or less). In contrast, a '164 sensor exposed to the same conditions darkens to a level so that within 8 days an electronic CO detector triggers an alarm response.

Although described above for the chemical reagent solutions used to make a CO sensor that is more responsive to CO at low to ambient humidity, the response threshold of sensors having an optimum response to CO under ambient to high relative humidity can also be controlled. This second sensor is typically red-orange in color and turns dark red to black upon exposure to CO above a specified low level threshold.

A chemically based CO sensor having an optimum response to CO at ambient to high relative humidity and a specific response threshold to low level CO (hereafter referred to as a "Formula #3 sensor") is made by impregnating a porous, semitransparent substrate with a chemical reagent containing at least one compound from each of the following groups: Group 1 comprising palladium compounds and their hydrates; Group 2 comprising molybdenum compounds and their hydrates; Group 3 comprising copper compounds and their hydrates; Group 4 comprising cyclodextrin molecular encapsulants; Group 5 comprising soluble chloride and bromide salts and their hydrates; Group 6 comprising halogenated acetic acid, and alkali metal and alkaline-earth metal salts of halogenated acetic acids; and Group 7 comprising a soluble metal trifluoroacetylacetonate.

Preferred Group 1 materials include those selected from the group consisting of palladium sulfate; palladium sulfite; palladium pyrosulfite; palladium chloride; palladium bromide; calcium, sodium and potassium salts of the tetrachloropallidate, bromotrichloropallidate, dibromodichloropallidate, tribromochloropallidate and tetrabromopallidate ions; and mixtures thereof. Preferred Group 2 materials include those selected from the group consisting of silicomolybdic acid; salts of silicomolybdic acid; molybdenum trioxide; ammonium molybdate; alkali or alkaline earth salts of molybdate anion; and mixtures thereof. Preferred Group 3 materials include those selected from the group consisting of copper sulfate; copper bromide; copper chloride; copper fluoride; copper iodide; copper trifluoroacetate; copper perchlorate; and mixtures thereof. Preferred Group 4 materials include those selected from the group consisting of α-cyclodextrin; β-cyclodextrin; modified β-cyclodextrin; γ-cyclodextrin; cyclodextrins having an internal cavity of at least 5 Å (5 x 10⁻¹⁰ m); and mixtures thereof. Preferred Group 5 materials include those selected from the group consisting of sodium, lithium, platinum, magnesium, calcium, strontium, beryllium, barrium, zinc and mixtures thereof. Preferred Group 6 materials include those selected from the group consisting of trichloroacetic acid; tribromoacetic acid; the sodium, potassium, calcium salts of trichloroacetate; the sodium, potassium, calcium salts of tribromoacetate; and mixtures thereof. Preferred Group 7 materials include those selected from the group consisting of copper, calcium, magnesium, sodium, potassium, lithium and mixtures thereof.

Components of the chemical reagent solution are present in the following mole ratio ranges to provide a chemical sensor having a desired controlled CO response threshold:
Group 2 : Group 1 = 0.283 to 0.472
Group 3 : Group 1 = 0.071 to 0.188
Group 4 : Group 1 = 0.149 to 0.249
Group 5 : Group 1 = 4.429 to 7.381
Group 6 : Group 1 = 0.279 to 0.464
Group 7 : Group 1 = 0.016 to 0.027

The porous, semitransparent substrate for the Formula #3 sensor is selected from the group consisting of: commercial silica gel desiccants in bead form (available form most major suppliers of silica gel); porous silicon dioxide, such as "GELSILs" (TM) available from Geltech of Alachua, Florida; and, other similar porous, semi-transparent monolithic substrates. The substrate preferably has an average surface pore size greater than about 15 nm (1.5 x 10⁻⁸ m) and preferably in the range of from about 15 to 100 nanometers (1.5 x 10⁻⁸ - 1.0 x 10⁻⁷ m), and more preferably an average surface pore size of approximately 30 nm (3.0 x 10⁻⁸m).

A preferred embodiment of the chemical reagent used to make the Formula #3 sensor is formed from an aqueous solution containing a mixture of chemical compounds selected from each of the following groups: the compound from Group 1 is a mixture palladium chloride and sodium tetrachloropallidate; the compound from Group 2 is silicomolybdic acid; the compound from Group 3 is copper chloride; the compound from Group 4 is a mixture of H-β -cyclodextrin and γ-cyclodextrin; the compound from Group 5 is a mixture of calcium bromide dihydrate and calcium chloride dihydrate; the compound from Group 6 is trichloroacetic acid; and the compound from Group 7 is copper bistrifluoroacetylacetonate.

As previously discussed above for Formula #1, the control of the response threshold of the Formula #3 sensors is a balance the desensitizing affects of Group 3 compounds and the competing sensitizing affects of Group 6 compounds. Therefore, although this formulation appears to be similar to that disclosed in the parent application, a point of invention lies within the molar ratio of compounds and the resulting threshold response. Accordingly, adherance to molar ratio ranges for the Groups of Formula #3 is key to the formulation of a sensor having both the predetermined CO response threshold and being capable of rapidly responding to higher CO concentrations.

The following example is of a Formula #3 sensor with a response threshold of 35 ppm, and is intended to illustrate and clarify a specific embodiment of the present invention and is not intended to limit the scope of the invention which is defined by the claims.

### EXAMPLE #3 - Preparation of Formula #3 Sensors Having a CO Response Threshold of 35 ppm

500 porous, silicon dioxide GELSIL(TM) discs (6.0 mm diameter, 2.9 mm thick) with a average surface pore size of 30.0 nm (3.0 x 10⁻⁸ m) available from Geltech of Alachua FL were soaked under ambient conditions for 20 hours in 75 ml of an aqueous chemical reagent solution containing the following: 0.0011 M sodium tetrachloropallidate (Group 1); 0.0226 M palladium chloride (Group 1); 0.0092 M silicomolybdic acid (Group 2); 0.0024 M copper chloride dihydrate (Group 3); 0.0032 M H-β -cyclodextrin (Group 4); 0.0011 M γ-cyclodextrin (Group 4); 0.1152 M calcium chloride dihydrate (Group 5); 0.0286 M calcium bromide dihydrate (Group 5); 0.009048 M trichloroacetic acid (Group 6); 0.0005 M copper bistrifluoroacetylacetonate (Group 7). The sensors were removed from the solution and were air dried for 56 hours in a relative humidity of about 50-60%, heated at 40°C for 24 hours and finally cooled to room temperature. Typically, sensors prepared in this manner are clear and have a red appearance.

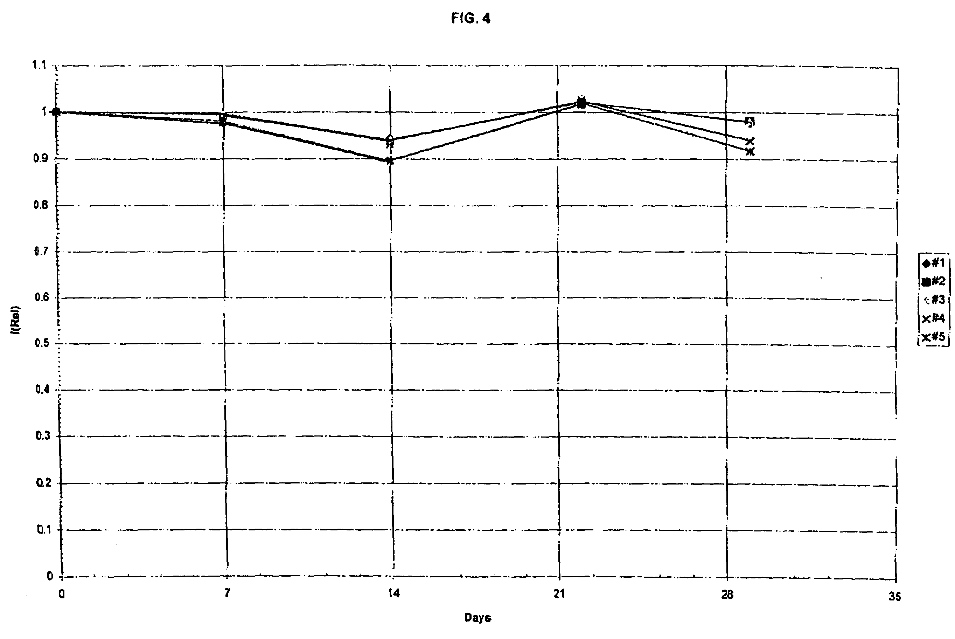

The response of Formula #3 sensors to 35 ppm CO at ambient relative humidity is given in Table 3 and experimental data is shown in graphical form in FIG. 4. All values are given relative to the initial intensity (Io) of the light attenuated by the sensor.

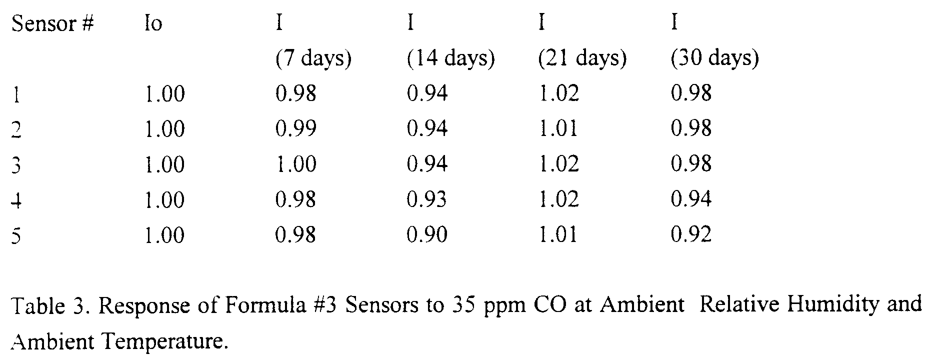

The above data shows that the Formula #3 sensor partially darkens during the 30 day exposure to 35 ppm CO. Even though the detected level of attenuated light varies over time, the intensity at any particular time is not reduced enough to trigger an alarm response in an electronic CO detector(e.g., I = 0.30 or less). In contrast, an ambient to high humidity sensor made in accordance with the parent application exposed to the same conditions darkens to a level so that within 8 days an electronic CO detector triggers an alarm response.

The optical combination of a sensor from Example #2 and Example #3, in accordance to the principles of the present invention, (hereafter referred to as "System A") gives a sensor system that has a response threshold of 35 ppm CO and remains responsive to higher concentration levels of CO under a wide range of atmospheric humidity conditions. Experimental data in support of this conclusion is given in the following tables.

The following data tables show the relative change in measured intensity of the light attenuated by the sensor system. The intensity at the beginning of the test (Io) was given a value of 1.00 and subsequent values were measured relative to Io. All measurements were conducted using standard and well known laboratory techniques.

### Measurement of the 35 ppm Response Threshold of System A.

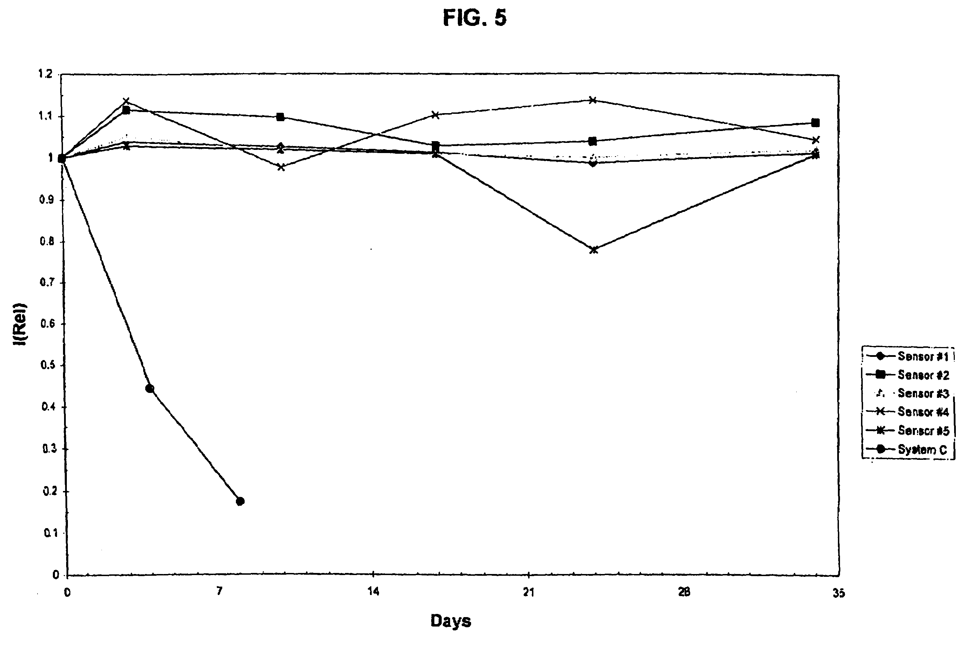

System A were exposed to 35 ppm CO under a controlled atmosphere maintained at 23°C and 52% relative humidity. The response of System A exposed to 35 ppm CO is given in Table 4 and experimental data is shown in graphical form in FIG. 5. All values are given relative to the initial intensity (Io) of the light attenuated by the sensor.

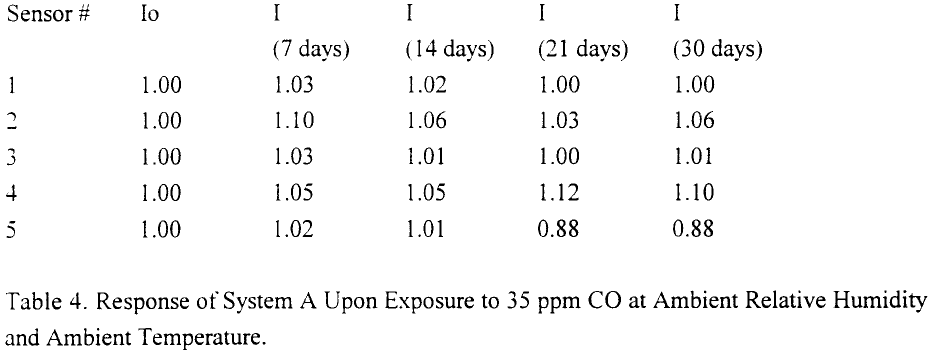

The above data shows that the sensors of System A partially darken upon exposure to 35 ppm CO. Even though the detected level of attenuated light varies over time, the intensity at any particular time is not reduced enough to trigger an alarm response in an electronic CO detector (e.g., I = 0.3 or less). Comparable sensor systems (hereafter referred to as "System C"), made without response threshold sensors, darken within 8 days to trigger an alarm response when exposed to the same conditions as is shown in FIG. 5.

### Measurement of the Response of System A Upon Exposure to 100 ppm CO for 90 Minutes at Ambient Relative Humidity and Ambient Temperature.

System A sensors were exposed to 100 ppm CO for 90 minutes under a controlled atmosphere maintained at 23°C and 52% relative humidity. The results of this test are given in Table 5.

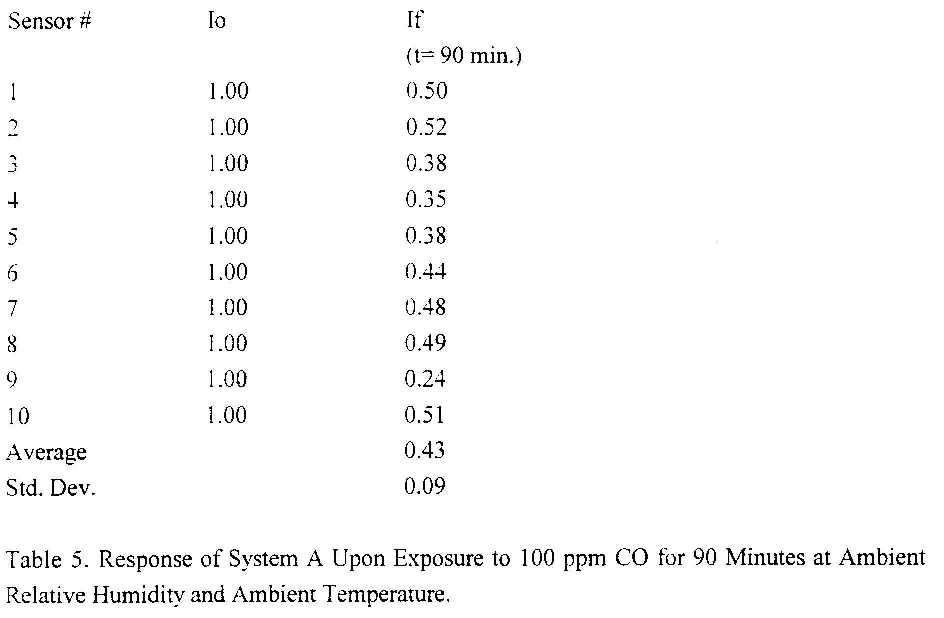

The above data shows that the System A sensor system darkens rapidly in response to 100 ppm CO under ambient conditions. The rapid change in the intensity of the detected attenuated light is sufficient to cause an electronic CO detector to trigger an alarm response (e.g., sounding of a horn, activation of a flashing light, activation of a safety shutoff valve, etc.). System A self-regenerates (i.e. becomes clear) within 24 hours exposure of System A to fresh air.

### Measurement of the Response of System A Upon Exposure to 100 ppm CO for 90 Minutes at Low Relative Humidity and Ambient Temperature.

System A sensors were exposed to 100 ppm CO for 90 minutes under a controlled atmosphere maintained at 23°C and 33% relative humidity. The results of this test are given in Table 6.

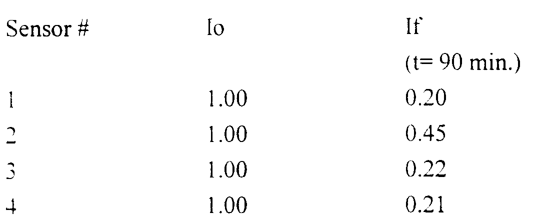

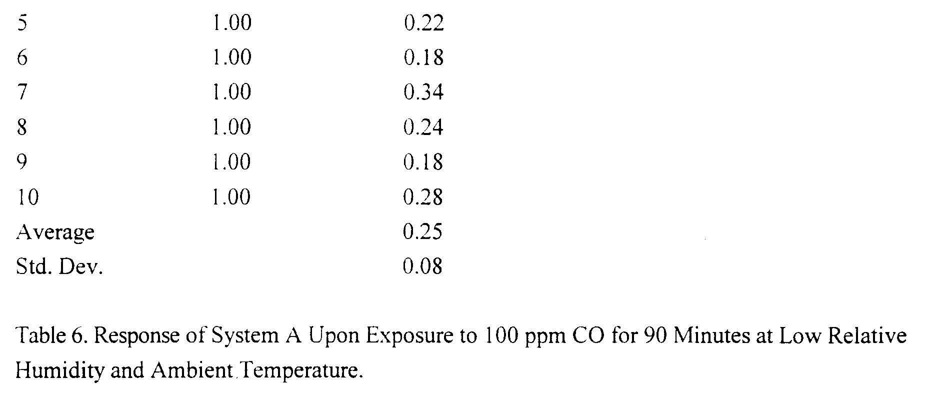

The above data shows that the System A sensor system darkens rapidly in response to 100 ppm CO under low relative humidity conditions. Either the difference in intensity or the rapid change in the intensity of the detected attenuated light is sufficient to cause an electronic CO detector to trigger an alarm response (e.g., sounding of a horn, activation of a flashing light, activation of a safety shutoff valve, etc.). System A self-regenerates (i.e. becomes clear) within 24 hours exposure of System A to fresh air.

### Measurement of the Response of System A Upon Exposure to 100 ppm CO for 90 Minutes at High Relative Humidity and Ambient Temperature.

System A sensors were exposed to 100 ppm CO for 90 minutes under a controlled atmosphere maintained at 23°C and 95% relative humidity. The results of this test are given in Table 7.

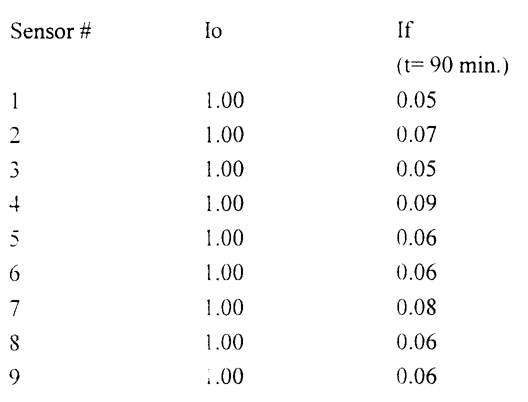

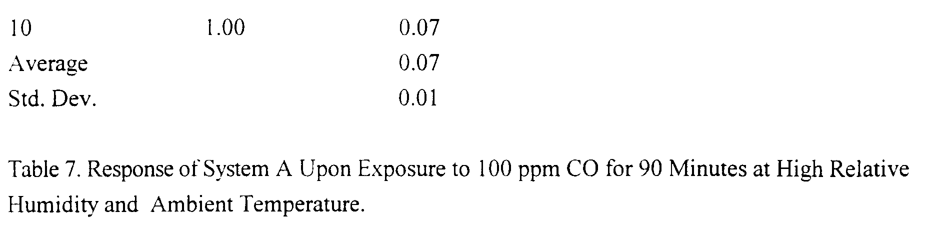

The above data shows that the System A sensor system darkens rapidly in response to 100 ppm CO under high relative humidity conditions. Either the difference in intensity or the rapid change in the intensity of the detected attenuated light is sufficient to cause an electronic CO detector to trigger an alarm response (e.g., sounding of a horn, activation of a flashing light, activation of a safety shutoff valve, etc.). System A self-regenerates (i.e. becomes clear) within 24 hours exposure of System A to fresh air.

We have found that by replacing or partially replacing the silicomolybdate anion by other heteropolymolybdate anions in the chemical reagent used to make the sensors(hereafter referred to as Formula #4), the response threshold of a CO sensor can be controlled. Especially useful are heteropoly acids, their salts and hydrates selected from compounds having the general formula H₄SiMo_{12-X}EₓO₄₀, where E is selected from the group consisting: chromium, vanadium, cobalt, manganese, iron, niobium, tantalum and tungsten and x has value between 2 and 6; silicomolybdic acid; silicotungstic acid; and mixtures thereof.

Without intending to be limited by any particular theory, we believe that the introduction of other metals into the structure of the silicomolybdic acid retards the reduction of the molybdenum containing species by the palladium catalyst. By making this change, the sensor does not darken as readily upon exposure to low level concentrations of CO. Therefore by carefully adjusting the amount and the redox properties of the molybdenum containing compounds, the response threshold of the Formula #4 sensors can be controlled.

Formula #4 sensors are made by the process previously described, that is by the impregnation of a porous, semi-transparent monolithic substrate with a CO sensitive chemical reagent. The substrate may be selected from the group comprising: silica gel beads; porous silicon dioxide, such as GELSIL(TM) available from GelTech of Alachua FL, or other similar porous, monolithic materials. It is desired that the substrate have an average surface pore size greater than about 15 nm (1.5 x 10⁻⁸ m), preferably in the range of from about 15 to 100 nm (2.0 x 10⁻⁸ - 1.0 x 10⁻⁷ m), and more preferably in the range from about 20 to 30 nanometers (2.0 x 10⁻⁸ - 3.0 x 10⁻⁸ m).

The Formula #4 impregnating chemical reagent is a mixture of chemical compounds containing at least one compound from each of the following groups: Group 1 comprising palladium compounds and their hydrates; Group 2 comprising molybdenum compounds and their hydrates; Group 3 comprising copper compounds and their hydrates; Group 4 comprising cyclodextrin molecular encapsulants; Group 5 comprising soluble chloride and bromide salts and their hydrates; Group 6 comprising halogenated acetic acid, and alkali metal and alkaline-earth metal salts of halogenated acetic acids; Group 7 comprising a soluble metal trifluoroacetylacetonate; and Group 8 comprising heteropolymetallic acids, their salts and hydrates.

Preferred Group 1 materials include those selected from the group consisting of palladium sulfate; palladium sulfite; palladium pyrosulfite; palladium chloride; palladium bromide; calcium, sodium and potassium salts of the tetrachloropallidate, bromotrichloropallidate; dibromodichloropallidate, tribromochloropallidate and tetrabromopallidate ions; and mixtures thereof. Preferred Group 2 materials include those selected from the group consisting of silicomolybdic acid; salts of silicomolybdic acid; molybdenum trioxide; ammonium molybdate; alkali or alkaline earth salts of molybdate anion; and mixtures thereof. Preferred Group 3 materials include those selected from the group consisting of copper sulfate; copper bromide; copper chloride; copper fluoride; copper iodide; copper trifluoroacetate; copper perchlorate; and mixtures thereof. Preferred Group 4 materials include those selected from the group consisting of α-cyclodextrin; β-cyclodextrin; modified β-cyclodextrin; γ-cyclodextrin; cyclodextrins having an internal cavity of at least 5 Å (5 x 10⁻¹⁰ m); and mixtures thereof. Preferred Group 5 materials include those selected from the group consisting of sodium, lithium, platinum, magnesium, calcium, strontium, beryllium, barium, zinc and mixtures thereof. Preferred Group 6 materials include those selected from the group consisting of trichloroacetic acid; tribromoacetic acid; the sodium, potassium, calcium salts of trichloroacetate; the sodium, potassium, calcium salts of tribromoacetate; and mixtures thereof. Preferred Group 7 materials include those selected from the group consisting of copper, calcium, magnesium, sodium, potassium, lithium and mixtures thereof. Preferred Group 8 materials include those selected from the group consisting of compounds having the formula H₄SiMo₁₂₋ₓEₓO₄₀, where E is selected from the group consisting: chromium, vanadium, cobalt, manganese, iron, niobium, tantalum and tungsten, and x has value between 2 and 6; silicotungstic acid; and mixtures thereof.

If the heteropolymolybdate compounds of Group 8 fully replace the silicomolybdate compounds of Group 2, components of the impregnating chemical reagent should be present in the following mole ratio ranges to provide a chemical sensor having a desired controlled CO response threshold:
Group 1 : Group 3 = 10.3 - 11.2
Group 2 : Group 3 = 0
Group 4 : Group 3 = 1.8 - 2.4
Group 5 : Group 3 = 98 - 110
Group 6 : Group 3 = 0.018 - 0.026
Group 7 : Group 3 = 3.2 - 4.6
Group 8 : Group 3 = 4.2 - 5.3

However, if the heteropolymolybdate compounds of Group 8 partially replace the silicomolybdate compounds of Group 2, components of the impregnating chemical reagent should be present in the following mole ratio ranges to provide a chemical sensor having a desired controlled CO response threshold:
Group 1 : Group 3 = 10.3 - 11.2
Group 2 : Group 3 = 1.60 - 4.25
Group 4 : Group 3 = 1.8 - 2.4
Group 5 : Group 3 = 98 - 110
Group 6 : Group 3 = 0.018 - 0.026
Group 7 : Group 3 = 3.2 - 4.6
Group 8 : Group 3 = 0.95 - 3.60

A preferred embodiment of formulation of chemical reagent used to make the Formula #4 sensor is formed from an aqueous solution containing a mixture of chemical compounds selected from each of the following groups: the compound from Group 1 is a mixture palladium chloride and sodium tetrachloropallidate; the compound from Group 2 is silicomolybdic acid; the compound from Group 3 is copper chloride; the compound from Group 4 is a mixture of H-β-cyclodextrin and γ-cyclodextrin; the compound from Group 5 is a mixture of calcium bromide dihydrate and calcium chloride dihydrate; the compound from Group 6 is trichloroacetic acid; the compound from Group 7 is copper bistrifluoroacetylacetonate; and the compound from Group 8 is silicodecamolybdobitungstic acid.

The following example is of a Formula #4 sensor with a response threshold of 35 ppm, and is intended to illustrate and clarify a specific embodiment of the present invention and is not intended to limit the scope of the invention which is defined by the claims.

### EXAMPLE #4 - Preparation of Formula #4 Sensors Having a CO Response Threshold of 35 ppm

200 porous, silicon dioxide GELSIL(TM) discs (6.0 mm diameter, 2.9 mm thick) with a average surface pore size of 27.2 nm (2.72 x 10⁻⁸ m) available from Geltech of Alachua FL were soaked under ambient conditions for 24 hours in 85 ml of an aqueous chemical reagent solution containing the following: 0.0023 M sodium tetrachloropallidate (Group 1); 0.0221 M palladium chloride (Group 1); 0.0055 M silicodecamolybdobitungstic acid (Group 8); 0.0055 M silicomolybdic acid (Group 2); 0.0023 M copper chloride dihydrate (Group 3); 0.0016 M H-β-cyclodextrin (Group 4); 0.0032 M γ -cyclodextrin (Group 4); and, 0.0913 M calcium chloride dihydrate (Group 5); 0.0285 M calcium bromide dihydrate (Group 5); 0.0091 M trichloroacetic acid (Group 6); 0.000052 M copper bistrifluoroacetylacetonate (Group 7). The sensors were removed from the solution and air dried for 24 hours under ambient conditions. They were then heated at 40°C for 24 hours and finally cooled to room temperature. Typically, Formula #4 sensors prepared in this manner are clear and have a red to maroon appearance.

The response of Formula #4 sensors to 35 ppm CO at ambient relative humidity is given in Table 8. All values are given relative to the initial intensity (Io) of the light attenuated by the sensor.

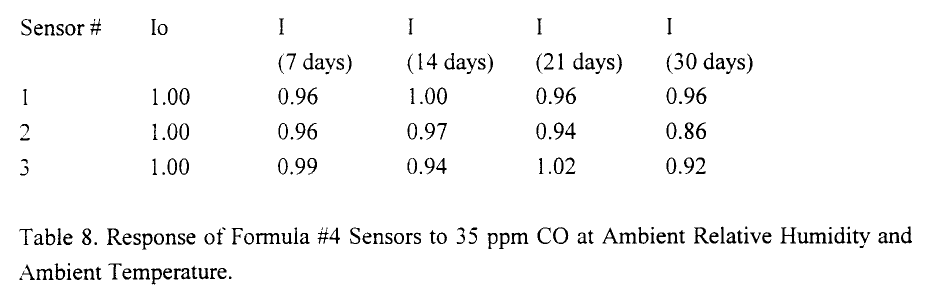

The above data shows that the Formula #4 sensor darkens only very slightly during the 30 day exposure to 35 ppm CO. Even though the level of attenuated light varies during the 30 day period, the intensity at any particular time is not reduced enough to trigger an alarm response in an electronic CO detector(e.g., I = 0.30 or less).

The response of the Formula #4 sensor upon exposure for 90 minutes to 100 ppm CO under a variety of relative humidity and temperature conditions is summarized in Table 4. The values of intensity given are the average of three or more readings. The final intensity reading (I(f)) was made after 90 minutes exposure to 100 ppm CO under the noted conditions and is relative to the initial reading (Io) made before the introduction of CO.

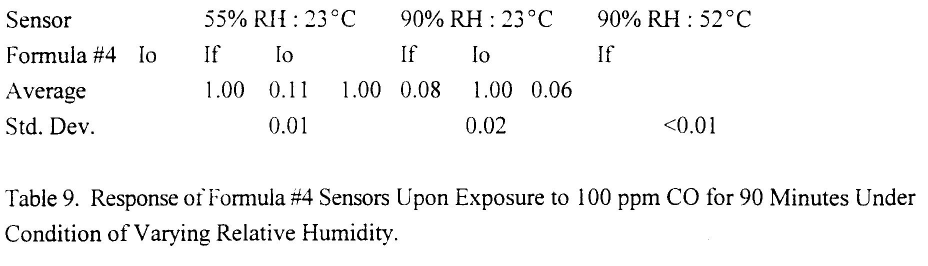

The above data shows that the Formula #4 sensor system darkens rapidly in response to 100 ppm CO under a wide range of relative humidity conditions. Either the difference in intensity or the rapid change in the intensity of the detected attenuated light is sufficient to cause an electronic CO detector to trigger an alarm response (e.g., sounding of a horn, activation of a flashing light, activation of a safety shutoff valve, etc.). The Formula #4 sensors self-regenerates (i.e. becomes clear) within 24 hours exposure to fresh air.

In addition to the above modifications of the formulation of the impregnating chemical reagent, we have found that chemical modification of the surface of the porous, semi-transparent substrate can also affect the response threshold of the sensor to low level CO. Therefore, it is within the scope of the present invention to chemically alter the surface structure and/or chemical composition of the sensor substrate to obtain sensors with a variety of low level CO response threshold levels.

One such modification of the substrate is the application of a molecularly thin metal oxide coating onto the surface of the substrate. It should be kept in mind that any such coating must be thick enough to increase the response threshold of the sensor to low concentrations of CO. However, the metal oxide coating must thin enough to remain sufficiently transmissive to light thereby allowing the detection of attenuated light by a light detection means. Therefore, there exists a balance of competing properties that is used to control of the response threshold of the sensors.

There are several different methods of applying molecularly thin metal oxide coatings onto sensor substrates. Such methods include: application of precursor chemicals from solution followed by heat treatment; application of precursor chemicals from solution followed by chemical treatment and heat treatment, such as that disclosed in U.S. Patent Application Serial Number 07\695,783, now abandoned; chemical vapor deposition; vacuum deposition; ion sputtering; and, other techniques known in the art. As described below, the preferred method is to apply the metal oxide layer using a precursor chemical contained in solution followed by heat treatment, however these other noted methods of applying molecularly thin layers of metal oxide will result in a sensor that is substantially similar and therefore is clearly within the scope of our invention.

We have found that the easiest, most economical, and therefore preferred, way to apply molecularly thin layers of metal oxide onto the surface of CO sensor substrates is to soak the substrates in a solution containing precursor chemicals followed by heat treatment. Metal oxides selected from the group of metals consisting: iron; copper; manganese; molybdenum, chromium; vanadium, cerium, niobium, tungsten and, mixtures thereof, have been found to be useful in controlling the response threshold of the sensor to low level concentrations of CO.

The following exemplary sensors having CO response thresholds of 35 ppm are intended to illustrate and clarify specific embodiments of the present invention and are not intended to limit the scope of the invention which is defined by the claims.

### EXAMPLE #5 - Preparation of Sensors Comprising Thin Metal Oxide Film and Having a CO Response Threshold of 35 ppm (hereinafter referred to as "Formula #5)

In a dry air atmosphere 50 GELSIL(TM) discs (6.0 mm diameter, 2.9 mm thick) with an average surface pore size of 20 nm (2.00 x 10⁻⁸ m) available from Geltech of Alachua FL were soaked in 10 ml of a dry isopropanol solution containing 0.031 M vanadium (tri-isopropoxide) oxide for 20 hours. The disks were removed from the solution and patted dry. The disks were removed from the dry conditions and transferred to a furnace which was slowly heated over 37 hours to a final temperature of 600°C. The final temperature was maintained for 15 hours after which the furnace was turned off and allowed to cool to room temperature. The surface modified sensors were then soaked for 24 hours in 5 ml of aqueous solution containing the following: 0.0023 M sodium tetrachloropallidate (Group 1); 0.022 M palladium chloride (Group 1); 0.0093 M silicomolybdic acid (Group 2); 0.0028 M copper chloride dihydrate (Group 3); 0.0026 M H-β-cyclodextrin (Group 4); 0.0013 M γ-cyclodextrin (Group 4); and, 0.11 M calcium chloride dihydrate (Group 5); and, 0.029 M calcium bromide dihydrate (Group 5). After soaking for 24 hours the impregnated disks were removed from the solution, blotted dry, allowed to dry at room temperature for 12 hours and finally heated for 24 hours at 40°C.

Upon exposure to 100 ppm CO the above sensors have an elongated response time when compared to sensors not have a treated substrate. That is to say the metal oxide treated sensors trigger an alarm response after 43 minutes while the untreated sensors trigger an alarm response within 22 minutes. The delayed response of the metal oxide sensors are therefore suitable for use in CO detectors that meet the UL 2034 standards in effect on October 1, 1995. The Formula #5 sensors self-regenerates (i.e. becomes clear) within 24 hours exposure to fresh air.

### EXAMPLE #6 - Preparation of Sensors Comprising Thin Metal Oxide Film and Having a CO Response Threshold of 35 ppm (hereinafter referred to as "Formula #6")

60 GELSIL(TM) discs (6.0 mm diameter, 2.9 mm thick) with an average surface pore size of 20 nm (2.00 x 10⁻⁸ m) available from Geltech of Alachua FL were soaked in 10 ml of an dry isopropanol solution containing 0.029 M coppermethoxyethoxide for 24 hours. The disks were removed from solution and patted dry. The disks were transferred to a furnace which was heated over 41 hours to a final temperature of 800°C. The final temperature was maintain for 15 hours after which the furnace was turned off and allowed to cool to room temperature. The surface modified disks appeared faint blue appearance after this treatment.

The surface modified sensors were then soaked for 24 hours in 5 ml of an aqueous chemical reagent solution containing the following: 0.0027 M sodium tetrachloropallidate (Group 1); 0.0384 M palladium chloride (Group 1); 0.011 M silicomolybdic acid (Group 2); 0.0013 M copper chloride dihydrate (Group 3); 0.0019 M β-cyclodextrin (Group 4); 0.0012 M γ-cyclodextrin (Group 4); and, 0.14 M calcium chloride dihydrate (Group 5). After soaking for 24 hours the impregnated disks were removed from the solution, blotted dry, allowed to dry at room temperature for 12 hours and finally heated for 24 hours at 40°C.

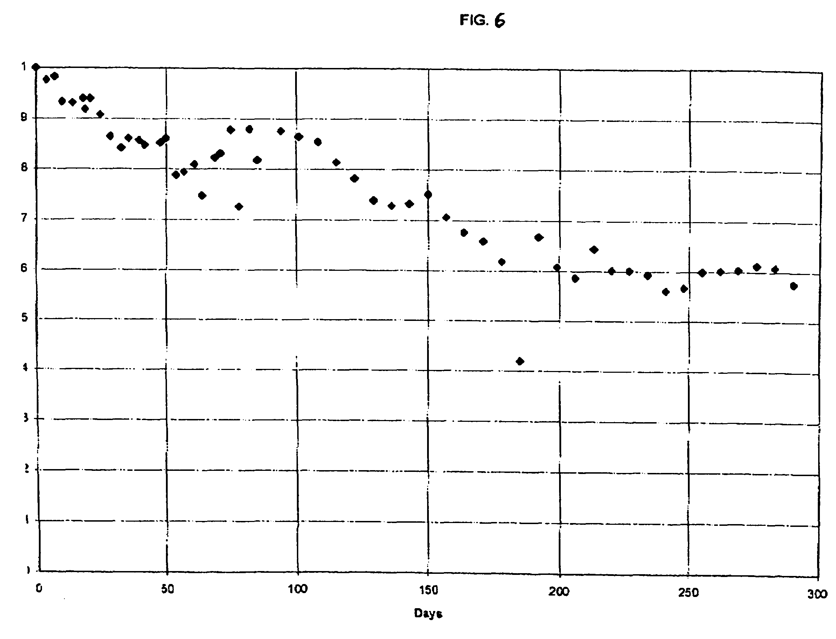

Sensors prepared in the above manner were exposed to 35 ppm of CO at room temperature and about 52% relative humidity for over 290 days. The average of five Forumual #6 sensors response is given in graphical form in FIG. 6 and a summary of the response of the sensor within the first thirty days is given in Table 11.

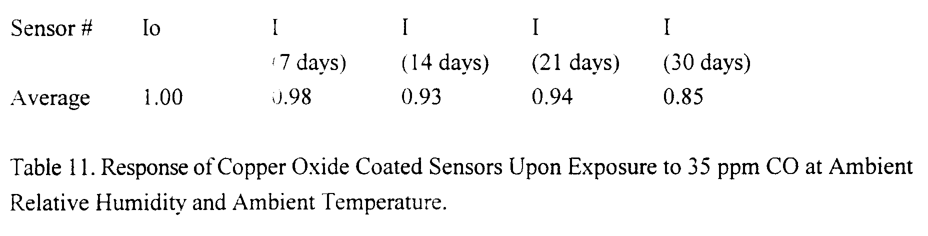

The above data shows that sensors having a copper oxide coated substrate darken only very slightly during a 30 day exposure to 35 ppm CO. Even though the level of attenuated light varies during the 290 day period, the intensity at any particular time is not reduced enough to trigger an alarm response in an electronic CO detector(e.g., I = 0.30 or less).

### EXAMPLE #7 - Preparation of Formula #2 Sensors with Copper Oxide Thin Film

100 porous silica GELSIL(TM) disks with an average surface pore size of 27.3 nm (2.73 x 10-8 m) available from GELTECH of Alachua, FL were immersed in an aqueous solution of 0.0001 M copper nitrate for 24 hours under ambient conditions. The discs were then removed from solution and allowed to dry for 18-24 hours. The disks were then exposed to the vapor of a 2.5 M ammonium hydroxide solution for 20-45 minutes until the disks became faint blue. The disks were then subjected to a heat treatment of at least 600°C for 12 hours and then cooled to room temperature to give copper oxide coated disks. The copper oxide treated disks were then immersed in 40 ml of an aqueous chemical reagent solution made in accordance to Formula #2 above for 24 hours. The impregnated sensors were removed from solution and dried in ambient air for 24 hours followed by drying in an oven at 40°C for 24 hours.

Sensors prepared in the above manner were exposed to 35 ppm of CO at room temperature and about 52% relative humidity for more than 30 days. The response of the sensor is given below in Table 12.

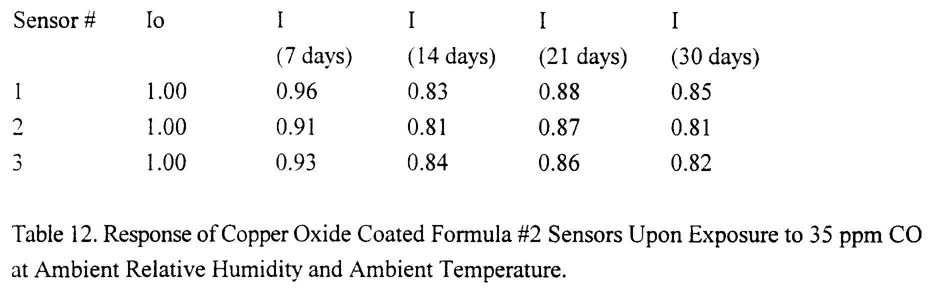

The above data shows that the Formula #2 sensors having a copper oxide coated substrate darken only very slightly during the 30 day exposure to 35 ppm CO. Even though the level of attenuated light varies during the 30 day period, the intensity at any particular time is not reduced enough to trigger an alarm response in an electronic CO detector(e.g., I = 0.30 or less).

The response of the above copper oxide surface treated sensors upon exposure for 90 minutes to 100 ppm CO under a variety of relative humidity and temperature conditions is summarized in Table 13. The values of intensity given are the average of three or more readings. The final intensity reading (I(f)) was made after 90 minutes exposure to 100 ppm CO under the noted conditions and is relative to the initial reading (Io) made before the introduction of CO.

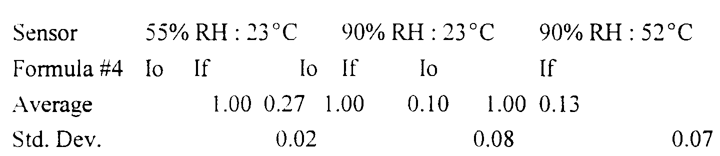

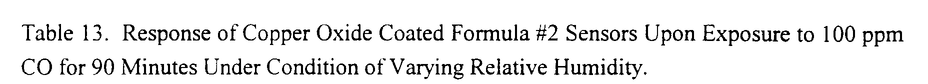

The above data shows that the copper oxide coated Formula #2 sensors darkens rapidly in response to 100 ppm CO under a wide range of relative humidity conditions. Either the difference in intensity or the rapid change in the intensity of the detected attenuated light is sufficient to cause an electronic CO detector to trigger an alarm response (e.g., sounding of a horn, activation of a flashing light, activation of a safety shutoff valve, etc.). The sensors self-regenerate (i.e. becomes clear) within 24 hours exposure to fresh air.

A number of different specific embodiments of chemical sensors having a predetermined response threshold have been specifically described and illustrated in relation to limited working embodiments for the purpose of clarity and illustration. The optical combination of two such specifically embodied sensors (Formula #2 and Formula #3 sensors) has been described as achieving the goals of the inventors, (i.e. response to CO under a wide range of relative humidity conditions and a predetermined response threshold). However, the interchange of sensors having similar characteristic, for example substitution of the Formula #3 sensor of System A with a Formula #4 sensor, will be apparent to one skilled in the art. Therefore such permutations in the sensors that constitute the sensor system are to be considered within the scope of the present invention.

Many other modifications and variations of the disclosed invention will be apparent to those skilled in the art. Therefore it is to be understood that, within the scope of the appended claims, CO sensors having a predetermined control response threshold and chemical sensor systems incorporating such sensors prepared according to principles of this invention may be embodied other than as specifically described herein.

## Claims

1. An optical sensor comprising:
a porous semi-transparent substrate that is at least partially transmissive to light; and
a self-regenerating chemical reagent impregnated into the substrate for detecting carbon monoxide, wherein the self-regenerating chemical reagent is formulated to regenerate itself for a period of at least one year under ambient conditions,
and wherein the sensor has a predetermined response threshold to carbon monoxide below which the sensor will not cause an alarm response.

2. An optical sensor as recited in claim 1 wherein the porous, semi-transparent substrate has a surface pore diameter in the range of from about 1.5 x 10⁻⁸ m to 1.0 x 10⁻⁷m.

3. An optical sensor as recited in claims 5 wherein the self-regenerating chemical reagent is a mixture comprising at least one compound selected from each of the following Groups:
Group 1 comprising palladium compounds and their hydrates;
Group 2 comprising molybdenum compounds and their hydrates;
Group 3 comprising copper salts and their hydrates;
Group 4 comprising cyclodextrin molecular encapsulants;
Group 5 comprising soluble chloride salts and their hydrates; and
Group 6 comprising halogenated acetic acids, and alkali metal and alkaline-earth metal salts of halogenated acetates.

4. An optical sensor as recited in claim 3 wherein:
the Group 1 compounds are selected from the group consisting of palladium chloride; palladium bromide; palladium sulfate; calcium, sodium and potassium salts of the tetrachloropallidate, bromotrichloropallidate, dibromodichloropallidate, tribromochloropallidate and tetrabromopallidate ions; and mixtures thereof;
the Group 2 compounds are selected from the group consisting of silicomolybdic acid; salts of silicomolybdic acid; molybdenum trioxide; ammonium molybdate; alkali or alkaline earth salts of molybdate anion; and, mixtures thereof;
the Group 3 compounds are selected from the group consisting of copper sulfate; copper nitrate; copper bromide; copper chloride; copper perchlorate; and mixtures thereof;
the Group 4 compounds are selected from the group consisting of α-cyclodextrin; β-cyclodextrin; modified β-cyclodextrin; γ-cyclodextrin; cyclodextrins having an internal cavity of at least 5 Å (5 x 10⁻¹⁰ m); and mixtures thereof;
the Group 5 compounds are selected from the group consisting of sodium, lithium, magnesium, calcium, and strontium ions, and mixtures thereof; and
the Group 6 compounds are selected from the group consisting of trichloroacetic acid; tribromoacetic acid; the sodium, potassium, and calcium salts of trichloroacetate; the sodium, potassium, and calcium salts of tribromoacetate; and mixtures thereof.

5. An optical sensor as recited in claim 2 wherein the self-regenerating chemical reagent is a mixture comprising at least one compound selected from each of the following Groups:
Group 1 comprising palladium compounds and their hydrates;
Group 2 comprising molybdenum compounds and their hydrates;
Group 3 comprising copper compounds and their hydrates;
Group 4 comprising cyclodextrin molecular encapsulants;
Group 5 comprising soluble chloride and bromide salts and their hydrates;
Group 6 comprising halogenated acetic acid, and alkali metal and alkaline-earth metal salts of halogenated acetates;
Group 7 comprising soluble metal halogenated acetylacetonate; and
Group 8 comprising heteropolymolybdenum acids, their salts and hydrates.

6. An optical sensor as recited in claim 5 wherein:
the Group 1 compounds are selected from the group consisting of palladium sulfate; palladium sulfite; palladium pyrosulfite; palladium chloride; palladium bromide; calcium, sodium and potassium salts of the tetrachloropallidate, bromotrichloropallidate, dibromodichloropallidate, tribromochloropallidate and tetrabromopallidate ions; and mixtures thereof
the Group 2 compounds are selected from the group consisting of silicomolybdic acid; alkali and alkaline earth salts of silicomolybdic acid, molybdenum trioxide; the alkali, alkaline earth and ammonium salts of the molybdate anion; and mixtures thereof;
the Group 3 compounds are selected from the group consisting of copper sulfate, copper bromide, copper chloride, copper fluoride, copper iodide, copper trifluoroacetate, copper perchlorate, and mixtures thereof;
the Group 4 compounds are selected from the group consisting of α-cyclodextrin; β-cyclodextrin; modified β-cyclodextrin; γ-cyclodextrin; cyclodextrins having an internal cavity of at least 5 Å (5 x 10⁻¹⁰ m); and mixtures thereof; and
the Group 5 compounds are selected from the group consisting of sodium, lithium, platinum, magnesium, calcium, strontium, beryllium, barium, zinc and mixtures thereof;
the Group 6 compounds are selected from the group consisting of trichloroacetic acid; tribromoacetic acid; the sodium, potassium, and calcium salts of trichloroacetate; the sodium, potassium, calcium salts of tribromoacetate; and mixtures thereof;
the Group 7 compounds are soluble metal trifluoroacetylacetonates selected from the group consisting of copper, calcium, magnesium, sodium, potassium, lithium, and mixtures thereof; and
the Group 8 compounds are selected from the group of compounds having the formula H₄SiMo₁₂₋ₓEₓO₄₀, where E is selected from the group comprising: chromium, vanadium, cobalt, manganese, iron, niobium, tantalum and tungsten, and x has value between 2 and 6; silicotungstic acid; and mixtures thereof.

7. An optical sensor comprising:
a porous, semitransparent substrate that is at least partially transmissive to light;
an optically thin film of metal oxide disposed onto a surface of the substrate; and
a chemical reagent impregnated into the substrate surface for detecting carbon monoxide to form the sensor, wherein the chemical reagent is formulated to regenerate itself for a period of at least one year under ambient conditions,
and wherein the sensor has a predetermined response threshold to carbon monoxide of greater than about 15 ppm, below which the sensor will not cause an alarm response.

8. A carbon monoxide sensing system comprising:
a first optical sensor;
a second optical sensor; and
a sensor housing adapted for optically combining the first sensor and the second sensor between a emitting means and a light detecting means,
wherein the carbon monoxide sensing system is adapted to detect carbon monoxide under low to high relative humidity conditions, is capable of regenerating itself for a period of at least one year under ambient conditions, and has a predetermined response threshold to carbon monoxide below which the sensor will not cause an alarm response.

9. A carbon monoxide sensing system as recited in claim 8, wherein the first optical sensor comprises:
a porous, semi-transparent substrate that is at least partially transparent to light; and
a first self-regenerating chemical reagent impregnated into the substrate, the first self-regenerating chemical reagent being formulated to detect carbon monoxide under low to ambient relative humidity conditions, wherein the second optical sensor comprises:
a porous, semi-transparent substrate that is at least partially transparent to light; and
a second self-regenerating chemical reagent for impregnated into the substrate, the second self-regenerating chemical reagent being formulated to detect carbon monoxide under ambient to high relative humidity conditions.

10. A carbon monoxide sensing system comprising:
a first optical sensor comprising a porous semi-transparent substrate impregnated with a first chemical reagent, wherein the first chemical reagent is formulated to both provide an optimum response to carbon monoxide under low to ambient relative humidity conditions, and to provide a carbon monoxide response threshold of greater than about 35 ppm;
a second optical sensor comprising a porous semi-transparent substrate impregnated with a second chemical reagent, wherein the second chemical reagent is formulated to both provide an optimum response to carbon monoxide under ambient to high relative humidity conditions, and to provide a carbon monoxide response threshold of greater than about 35 ppm;
a sensing system housing adapted for optically combining the first sensor and the second sensor between a emitting means and a light detecting means,
wherein the carbon monoxide sensing system is capable of regenerating itself for a period of at least one year under ambient conditions.
